(19) Europäisches Patentamt
European Patent Office
Office européen des brevets

(11) Numéro de publication: **0 451 007 B1**

(12) # FASCICULE DE BREVET EUROPEEN

(45) Date de publication du fascicule du brevet: **08.11.95**

(51) Int. Cl.[6]: **C07H 15/203**, C07C 323/65, A61K 31/70

(21) Numéro de dépôt: **91400750.5**

(22) Date de dépôt: **20.03.91**

Le dossier contient des informations techniques présentées postérieurement au dépôt de la demande et ne figurant pas dans le présent fascicule.

(54) **Sulfonyl-phényl-beta-D-thioxylosides, leur procédé de préparation et leur utilisation en thérapeutique.**

(30) Priorité: **02.04.90 FR 9004173**

(43) Date de publication de la demande: **09.10.91 Bulletin 91/41**

(45) Mention de la délivrance du brevet: **08.11.95 Bulletin 95/45**

(84) Etats contractants désignés: **AT BE CH DE DK ES FR GB GR IT LI LU NL SE**

(56) Documents cités: **EP-A- 0 290 321**

(73) Titulaire: **FOURNIER INDUSTRIE ET SANTE**
**38, avenue Hoche**
**F-75008 Paris (FR)**

(72) Inventeur: **Samreth, Soth**
**2, rue de la Deuxième Escadre**
**F-21600 Longvic (FR)**
Inventeur: **Renaut, Patrice**
**3, ruelle de Plombières,**
**Hauteville - Lès Dijon**
**F-21121 Fontaine Lès Dijon (FR)**
Inventeur: **Bajgrowicz, Jerzy**
**Résidence Altirama**
**Avenue Frédéric Mistral,**
**F-06130 Grasse (FR)**
Inventeur: **Millet, Jean**
**15, rue du Tremblois,**
**Corcelles les Citeux**
**F-21910 Saulon la Rue (FR)**

(74) Mandataire: **Clisci, Serge et al**
**S.A. FEDIT-LORIOT**
**CONSEILS EN PROPRIETE INDUSTRIELLE**
**38, avenue Hoche**
**F-75008 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne, en tant que produits industriels nouveaux, les composés sulfonyl-phényl-β-D-thioxylosides de formule I ci-dessous. Elle concerne également leur procédé de préparation et leur utilisation en thérapeutique, en tant qu'agents antithrombotiques, notamment antithrombotiques veineux.

On a déjà proposé dans EP-B-0 051 023 des dérivés de benzoyl-phényl-osides et d'α-hydroxybenzyl-phényl-osides en tant qu'agents anti-ulcéreux, anti-agrégants plaquettaires, antithrombotiques et oxygénateurs cérébraux.

On connait également de EP-A-0 133 103 des benzyl-phényl-osides utiles en tant qu'agents hypocholestérolémiants et hypolipidémiants, certains de ces composés, en particulier le produit de l'exemple 1, présentant en outre des effets antithrombotiques.

On connait enfin de EP-A-0 290 321 des dérivés de benzoyl-phényl-thioxylose, α-hydroxybenzyl-phényl-thioxylose et benzyl-phényl-thioxylose proposés en tant qu'agents antithrombotiques.

On vient à présent de trouver que les composés sulfonyl-phényl-β-D-thioxylosides selon l'invention, structurellement différents des produits connus de l'art antérieur, sont utiles dans le traitement et la prévention des maladies liées aux troubles circulatoires, notamment en tant qu'agents antithrombotiques veineux.

Les nouveaux produits selon l'invention sont caractérisés en ce qu'ils sont choisis parmi l'ensemble constitué par les sulfonyl-phényl-β-D-thioxylosides de formule :

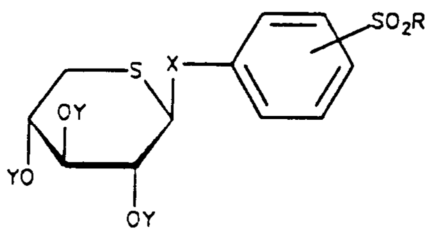

(I)

dans laquelle :

- X représente un atome de soufre ou un atome d'oxygène,
- R représente un groupe alkyle en $C_1$-$C_4$, un groupe amino substitué $NR_1R_2$, où $R_1$ et $R_2$, identiques ou différents, représentent chacun un groupe alkyle en $C_1$-$C_4$, $R_1$ et $R_2$ considérés ensemble pouvant former avec l'atome d'azote auquel ils sont liés un groupe pipéridinyle ou morpholinyle, ou un groupe phényle éventuellement substitué en position para par un groupe cyano ou par un atome d'halogène; et,
- Y représente l'atome d'hydrogène ou un groupe acyle aliphatique.

Parmi les groupes acyles aliphatiques qui conviennent selon l'invention, on peut mentionner ceux qui renferment au total 2 à 5 atomes de carbone, le groupe acyle aliphatique préféré étant $CH_3CO$.

Par groupe alkyle en $C_1$-$C_4$, on entend ici un reste hydrocarboné, ramifié ou linéaire, contenant 1 à 4 atomes de carbone, le groupe alkyle préféré étant le groupe méthyle.

Par atome d'halogène, on entend ici les atomes de chlore, de fluor et de brome, l'atome d'halogène préféré étant l'atome de fluor.

Selon l'invention on préfère les composés de formule I dans laquelle X représente l'atome de soufre.

Les composés de formule I et les composés acylés correspondants peuvent être préparés selon une réaction de glycosylation caractérisée en ce que :

(i) on fait réagir un composé de formule :

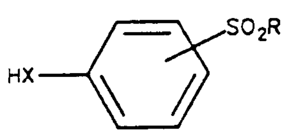

(II)

où X et R sont définis comme ci-dessus,

avec un dérivé du thioxylose choisi parmi l'ensemble comprenant :

(i) les halogénures d'acylthioxylosyle de formule :

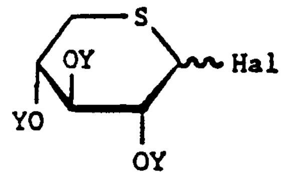

(III)

(ii) les thioxyloses peracylés de formule :

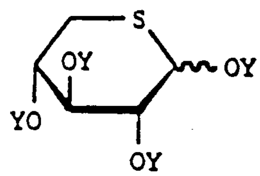

(IV)

et,

(iii) les trichloracétimidates d'acylthioxylosyle de formule :

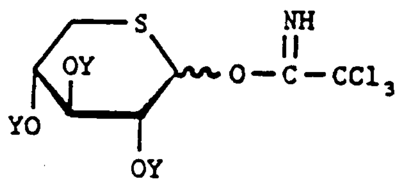

(V)

dans lesquelles Hal représente un atome d'halogène tel que Cl ou Br, l'atome de brome étant ici l'atome d'halogène préféré, et Y représente un groupe acyle, notamment un groupe acyle aliphatique comprenant un nombre total d'atomes de carbone de 2 à 5 et de préférence le groupe acétyle, dans un solvant inerte, à raison de 1 mole de II pour environ 0,6 à 1,2 moles de composé III, IV ou V, notamment en présence d'un accepteur d'acide et/ou d'un acide de Lewis, et,

(ii) si nécessaire, on soumet le composé de formule I ainsi obtenu où Y est un groupe acyle en $C_2$-$C_5$ à une réaction de désacylation à une température comprise entre 0 °C et la température de reflux du milieu réactionnel, dans un alcool inférieur en $C_1$-$C_4$, de préférence le méthanol, en présence d'un alcoolate métallique, de préférence le méthylate de magnésium ou le méthylate de sodium, pour obtenir un composé de formule I où Y est H.

Les composés III, IV et V peuvent être de configuration $\alpha$ ou $\beta$ ou sous forme d'un mélange anomérique des deux configurations.

Les réactions de glycosylation des composés de formule II ont été conduites, soit au départ du composé III en présence d'un catalyseur comme les sels ou oxydes d'argent, de mercure ou de zinc, soit au départ du composé V en présence d'un acide de Lewis, notamment l'éthérate de trifluorure de bore ou le chlorure de zinc, soit encore au départ du composé IV en présence d'un acide de Lewis.

Selon un mode préféré de mise en oeuvre de l'invention, on préconise de condenser 1 mole du composé de formule II avec environ 1,1 à 1,2 moles d'halogénure d'acylthioxylosyle III dans un solvant inerte choisi parmi les solvants polaires ou apolaires (comme par exemple le diméthylformamide, le tétrahydrofuranne, le dioxanne, l'acétonitrile, le nitrométhane, le benzène, le toluène, les xylènes et leurs mélanges), en présence de cyanure mercurique.

On utilise avantageusement le bromure de 2,3,4-tri-O-acétyl-5-thio-$\alpha$-D-xylopyranosyle dans un mélange benzène-nitrométhane (1/1) v:v, en présence de 1,1 à 1,3 moles de cyanure mercurique, à une température comprise entre 0 °C et la température de reflux du milieu réactionnel, de préférence à environ 40-50 °C, pendant 1 à 4 heures, de préférence pendant environ 2 heures.

Selon un deuxième mode préféré de mise en oeuvre de l'invention, on préconise de condenser 1 mole du composé de formule II avec environ 1,1 à 1,2 moles d'halogénure d'acylthioxylosyle III dans un solvant inerte (comme par exemple le dichlorométhane ou l'acétonitrile) en présence d'imidazolate d'argent et de

chlorure de zinc.

On utilisera avantageusement le bromure de 2,3,4-tri-O-acétyl-5-thio-D-xylopyranosyle dans du dichlorométhane ou un mélange dichlorométhane/acétonitrile en présence de 1,5 à 1,7 moles d'imidazolate d'argent et de 2 à 2,2 moles de chlorure de zinc, à une température comprise entre 0 °C et la température de reflux du milieu réactionnel, de préférence à environ 40-60 °C, pendant 24 à 48 heures.

Selon un troisième mode préféré de mise en oeuvre de l'invention, on préconise de condenser 1 mole du composé de formule II avec environ 0,6 à 1 mole d'halogénure d'acylthioxylosyle III dans un solvant inerte (comme par exemple le toluène et/ou l'acétonitrile) en présence d'oxyde de zinc.

On utilisera avantageusement le bromure de 2,3,4-tri-O-acétyl-5-thio-D-xylopyranosyle dans un mélange toluène/acétonitrile, en présence de 0,5 à 1,2 moles d'oxyde de zinc, à une température comprise entre la température ambiante et la température de reflux du milieu réactionnel, de préférence à environ 40-60 °C, pendant 18 à 48 heures.

Selon un quatrième mode préféré de mise en oeuvre de l'invention, on préconise de condenser 1 mole du composé de formule II, avec environ 1,1 à 1,3 moles de trichloracétimidate d'acylthioxylosyle dans un solvant inerte (comme par exemple le dichlorométhane ou l'acétonitrile) en présence d'éthérate de trifluorure de bore ou de chlorure de zinc.

On utilisera avantageusement le trichloracétimidate de 2,3,4-tri-O-acétyl-5-thio-α-D-xylopyranosyle dans du dichlorométhane, en présence de 0,1 à 0,4 mole d'éthérate de trifluorure de bore en solution dans du dichlorométhane ou de l'acétonitrile, ou en présence de chlorure de zinc, à une température comprise entre - 40 °C et la température ambiante (15-25 °C), de préférence à environ - 20 °C à 0 °C, pendant 1 à 5 heures.

La réaction de glycosylation conduit dans tous les cas à un mélange des isomères de configuration $\alpha$ et $\beta$ en proportions variables.

L'isomère de configuration $\beta$ est isolé selon les méthodes connues de l'homme de l'art, comme par exemple la cristallisation fractionnée ou la chromatographie, notamment la "flash chromatography" [i.e. chromatographie sur colonne de silice et sous pression selon la technique décrite par W.C. STILL et al. dans J. Org. Chem. (1978), 42 (N° 14) 2923].

Les dérivés obtenus sont soumis, le cas échéant, à une désacylation, plus particulièrement à une désacétylation, qui est réalisée à une température comprise entre 0 °C et la température de reflux du milieu réactionnel dans un alcool inférieur en $C_1$-$C_4$, en présence de l'alcoolate métallique correspondant. De préférence, on choisira le méthanol comme alcool inférieur et le méthanolate de sodium ou de magnésium comme alcoolate métallique.

La réaction de désacylation peut éventuellement être conduite après glycosylation sans isoler le composé acylé intermédiaire formé.

On peut également réaliser la réaction de désacylation par voie enzymatique, par exemple par action de l'estérase de foie de porc.

Pour accéder aux composés intermédiaires de formule II où X = S on préconise :

(i) de condenser, en milieu basique fort, le chlorure de diméthyl-amino-thiocarbamoyle, de formule :

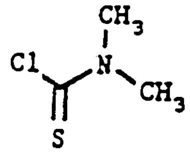

(VI)

sur un composé de formule :

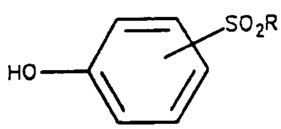

(IIa)

où R a la signification indiquée ci-dessus, pour obtenir un composé de formule :

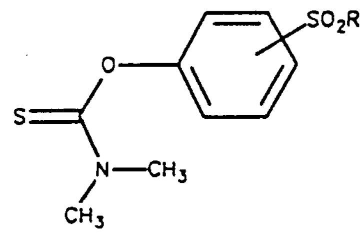

(VII)

où R a la signification indiquée ci-dessus,
(ii) soumettre le composé de formule VII ainsi obtenu à un réarrangement de Newmann (J. Org. Chem. (1966) 31, p 3980), par chauffage, pour obtenir un composé de formule :

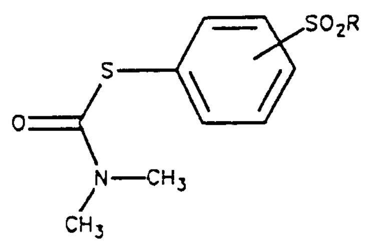

(VIII)

où R a la signification indiquée ci-dessus,
(iii) traiter le composé de formule VIII ainsi obtenu par un alcoolate métallique, de préférence le méthanolate de sodium ou de magnésium, dans un alcool inférieur en $C_1$-$C_4$, de préférence le méthanol, le diméthylformamide ou le dioxanne pour obtenir un composé de formule II où X = S.

On peut également accéder aux composés intermédiaires de formule II où X = S par substitution nucléophile d'un composé halogénobenzène approprié selon la méthode décrite par L. TESTAFERRI dans Tetrahedron Letters Vol. 21 p.3099-3100 (1980).

Certains composés intermédiaires de formule II où X = S sont des composés nouveaux.

Les composés de formule :

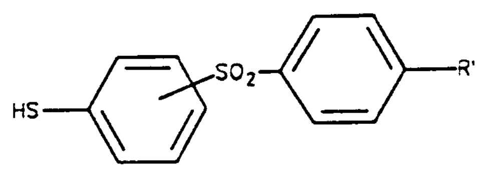

dans laquelle :

- R′ représente un atome de fluor, un atome de brome ou un groupe cyano, constituent donc un des objets de l'invention.

Selon l'invention, on propose une composition thérapeutique caractérisée en ce qu'elle renferme, en association avec un excipient physiologiquement acceptable, au moins un composé choisi parmi l'ensemble constitué par les produits de formule I. Bien entendu, dans une telle composition l'ingrédient actif intervient en quantité thérapeutiquement efficace.

Les composés de formule I sont utiles en thérapeutique en tant qu'agents antithrombotiques. Ils sont notamment utiles dans la prévention et le traitement des troubles de la circulation veineuse.

Selon l'invention, on préconise l'utilisation d'une substance appartenant à l'ensemble des composés de formule I pour l'obtention d'un médicament antithrombotique destiné à une utilisation thérapeutique vis-vis des troubles de la circulation veineuse.

D'autres caractéristiques et avantages de l'invention seront mieux compris à la lecture qui va suivre, d'exemples de préparation, nullement limitatifs, donnés à titre d'illustration, et de résultats d'essais pharmacologiques.

Dans les exemples de préparation qui suivent, les composés ont été nommés en précisant la configuration α ou β quand ladite configuration a été déterminée. Lorsque la configuration n'est pas indiquée, cela signifie que le produit correspondant est un mélange anomérique des configurations α et β dans des proportions qui n'ont pas été déterminées.

**PREPARATION I**

I a) Obtention du 4-(phénylsulfonyl)-benzènethiol.

1,25 g de thiométhanolate de sodium sont additionnés sous atmosphère d'azote à une solution de 15 g (0,0593 mole) de 1-chloro-4-(phénylsulfonyl)-benzène dans 150 ml d'hexaméthyl phosphoramide. Le mélange obtenu est chauffé pendant 4 heures à 100 °C puis, après refroidissement, hydrolysé sur un mélange glace/eau. Le milieu réactionnel obtenu est extrait à l'acétate d'éthyle puis la phase aqueuse obtenue est versée sur une solution d'acide chlorhydrique 4N à 0 °C. Le produit formé précipite. Après filtration, lavage à l'eau jusqu'à pH neutre et séchage, on obtient 13,44 g (rendement : 90,5 %) du produit attendu.
F = 117 °C

I b) Obtention du 4-(phénylsulfonyl)phényl 2,3,4-tri-O-acétyl-1,5-dithio-β-D-xylopyranoside (exemple 1)

10,6 g de cyanure mercurique ($Hg(CN)_2$) sont additionnés sous azote à une solution de 10 g (0,0399 mole) de 1-mercapto-4-(phénylsulfonyl)benzène dans 300 ml d'un mélange toluène/nitrométhane 1/1 (v/v), puis le mélange obtenu est agité 1 heure à 40-45 °C. On observe une précipitation. 17,7 g (0,0498 mole) de 2,3,4-tri-O-acétyl-1-bromo-5-thio-β-D-xylopyranoside sont ensuite ajoutés au mélange. Après 3,5 h d'agitation à 40-45 °C le milieu réactionnel devient limpide. La phase organique obtenue après refroidissement est lavée successivement à l'aide d'une solution d'acide chlorhydrique 1N à 0 °C, d'une solution de soude 1N à 0 °C, à l'eau puis à l'aide d'une solution saturée de chlorure de sodium. On obtient après évaporation des solvants 24 g d'une mousse jaune qui cristallise par addition d'éther. On obtient finalement 8,6 g du produit attendu (rendement : 41 %).
F = 159 °C
$[\alpha]_D^{23}$ = + 58,2 ° (c = 0,5 ; $CHCl_3$)

I c) Obtention du 4-(phénylsulfonyl)phényl 1,5-dithio-β-D-xylopyranoside (exemple 2)

0,35 $cm^3$ de méthylate de sodium en solution dans le méthanol (3,5 molaire) sont ajoutés à une suspension de 6,5 g de 4-(phénylsulfonyl)phényl 2,3,4-tri-O-acétyl 1,5-dithio-β-D-xylopyranoside dans 150 ml de méthanol. Le mélange obtenu est agité à température ambiante pendant 1,5 h sous azote, puis 250 $cm^3$ de tétrahydrofurane sont ajoutés. On obtient une solution limpide avant d'additionner de la résine Amberlite[R] IR 120 $H^+$ jusqu'à pH 6. On filtre et évapore les solvants sous pression réduite. Après recristallisation dans un mélange méthanol/eau 50/50 (v/v) puis dissolution dans un mélange éthanol/eau 50/50 (v/v) et lyophilisation, on obtient 3,3 g du produit attendu (rendement : 67 %).
F = plage de fusion de 85 °C à 97 °C
$[\alpha]_D^{23}$ = + 44,8 ° (c = 0,42 ; diméthylsulfoxyde)

**PREPARATION II**

II a) Obtention du 1-bromo-2-(méthylsulfonyl)-benzène

Une solution de 5 g (0,0246 mole) de 1-bromo-2-(méthylthio)-benzène dans 10 ml de méthanol est refroidie à 0 °C sous atmosphère d'azote puis 1,27 g d'acide 3-chloroperoxybenzoïque (MCPBA) à 50 % sont ajoutés. L'agitation à 0 °C est maintenue pendant 45 minutes puis 6 g de fluorure de potassium sont additionnés et l'hydrolyse est poursuivie pendant 12 heures. Le milieu obtenu est filtré sur Célite[R], puis le produit obtenu après évaporation des solvants est purifié par "flash chromatography" en éluant avec un mélange toluène/acétate d'éthyle 95/5 (v/v). On obtient 5,63 g (rendement : 97 %) du produit attendu.
F = 98 °C

II b) Obtention du 2-(méthylsulfonyl-)-benzènethiol

En opérant de façon analogue à la préparation I a) on obtient le produit attendu.
F = 57 °C.

II c) Obtention du 2-(méthylsulfonyl) phényl 2,3,4-tri-O-acétyl-1,5-dithio-β-D-xylopyranoside (exemple 3)

8,43 g (0,0251 mole) de 1-bromo-2,3,4-tri-O-acétyl-5-thio-β-D-xylopyranoside sont ajoutés à un mélange de 4,3 g (0,0228 mole) de 1-mercapto-2-(méthylsulfonyl)-benzène et de 1,95 g d'oxyde de zinc dans 90 ml de toluène/acétonitrile 1/1 (v/v) et le mélange obtenu est chauffé à 45 °C pendant 2 heures. Le milieu obtenu est filtré sur Célite$^R$ puis la phase organique est lavée au moyen d'une solution d'HCl 1N, d'une solution de soude 1N, puis à l'eau jusqu'à pH neutre. Après évaporation des solvants sous pression réduite on obtient une huile qui cristallise par addition d'éther. Les 5,33 g de produit cristallisé obtenus sont ensuite purifiés par "flash chromatography" en éluant avec un mélange toluène/acétate d'éthyle 8/2 (v/v). On obtient 4,35 g (rendement : 41 %) du produit attendu.
F = 209 °C
$[\alpha]_D^{20}$ = + 38,4 ° (c = 0,5 ; $CHCl_3$)

II d) Obtention du 2-(méthylsulfonyl)phényl 1,5-dithio-β-D-xylopyranoside (exemple 4)

En opérant de façon analogue à la préparation I c) on obtient le produit attendu.
F = 139 °C
$[\alpha]_D^{20}$ = + 38,8 ° (c = 0,5 ; $CH_3OH$)

**PREPARATION III**

III a) Obtention du 4-(méthylsulfonyl)phényl 2,3,4-tri-O-acétyl-1,5-dithio-β-D-xylopyranoside (exemple 5)

En opérant de façon analogue à la préparation II c) on obtient une huile incolore qui cristallise dans l'éther.
F = 105-110 °C
$[\alpha]_D^{20}$ = + 71 ° (c = 0,5 ; $CHCl_3$)

III b) Obtention du 4-(méthylsulfonyl)phényl 1,5-dithio-β-D-xylopyranoside (exemple 6)

En opérant de façon analogue à la préparation I c) on obtient le produit attendu après recristallisation dans un mélange méthanol/éthanol.
F > 250 °C
$[\alpha]_D^{20}$ = + 19,2 ° (c = 0,5 ; $CH_2Cl_2$;$CH_3OH$ 1/1 (v/v))

**PREPARATION IV**

IV a) Obtention du 4-(méthylsulfonyl)phényl2,3,4-tri-O-acétyl-5-thio-β-D-xylopyranoside (exemple 7)

Un mélange de 2 g (0,012 mole) de 1-hydroxy-4-(méthylsulfonyl)benzène, de 3,17 g de chlorure de zinc ($ZnCl_2$), de 4,5 g (0,013 mole) de 1-bromo-2,3,4-tri-O-acétyl-5-thio-β-D-xylopyranoside et de 3,1 g (0,0177 mole) d'imidazolate d'argent ($C_3H_3AgN_2$) dans 70 ml de chlorure de méthylène est chauffé pendant 20 heures à 50 °C. Après refroidissement le milieu réactionnel est filtré puis la phase organique obtenue est lavée successivement au moyen d'une solution d'acide chlorhydrique 1N, d'eau et d'une solution de soude 1N puis le solvant est évaporé sous pression réduite. On obtient après purification par "flash chromatography" en éluant avec un mélange toluène/acétate d'éthyle 3/1 (v/v) puis précipitation dans l'éther éthylique 1,1 g du produit attendu (rendement : 21,2 %).
F = 168 °C
$[\alpha]_D^{22}$ = - 75 ° (c = 0,6 ; $CHCl_3$)

IV b) Obtention du 4-(méthylsulfonyl)phényl 5-thio-β-D-xylopyranoside (exemple 8)

En opérant de façon analogue à la préparation I c) on obtient le produit attendu après lyophilisation.
F = 180 °C
$[\alpha]_D^{22}$ = - 77,2 ° (c = 0,5 ; $CH_3OH$)

**PREPARATION V**

V a) Obtention du 4-(éthylsulfonyl)-benzènethiol

En opérant de façon analogue à la préparation I a) on obtient le produit attendu sous forme d'huile.
$n_D^{24}$ = 1,5891

V b) Obtention du 4-(éthylsulfonyl)phényl 2,3,4-tri-O-acétyl-1,5-dithio-β-D-xylopyranoside (exemple 9)

En opérant de façon analogue à la préparation I b) on obtient le produit attendu.
F = 136-137 °C
$[\alpha]_D^{23}$ = + 36,7 ° (c = 0,45 ; $CHCl_3$)

V c) Obtention du 4-(éthylsulfonyl)phényl 1,5-dithio-β-D-xylopyranoside (exemple 10)

En opérant de façon analogue à la préparation I c) on obtient le produit attendu.
F = 130-135 °C
$[\alpha]_D^{23}$ = + 26,8 ° (c = 0,485 ; méthanol)

**PREPARATION VI**

VI a) Obtention du 3-(méthylsulfonyl)phényl 2,3,4-tri-O-acétyl-1,5-dithio-β-D-xylopyranoside (exemple 11)

En opérant de façon analogue à la préparation II c) on obtient le produit attendu.
F = 147-150 °C
$[\alpha]_D^{21}$ = - 10,5 ° (c = 0,3 ; $CHCl_3$)

VI b) Obtention du 3-(méthylsulfonyl)phényl 1,5-dithio-β-D-xylopyranoside (exemple 12)

En opérant de façon analogue à la préparation I c) on obtient le produit attendu.
F = 169-172 °C
$[\alpha]_D^{22}$ = - 12,2 ° (c = 0,45 ; diméthylsulfoxyde)

**PREPARATION VII**

VII a) Obtention du 4-[(4-fluorophényl)sulphonyl] benzènethiol

A une suspension de 1 g (0,00398 mole) de 4-[(4-fluorophényl)sulfonyl]-benzènamine dans une solution d'acide chlorhydrique (1,68 ml d'acide chlorhydrique concentré dans 5 ml d'eau) à -5 °C, on ajoute une solution de nitrite de sodium (302 mg dans 1 ml d'eau). Après 5 minutes sous agitation, cette solution est ajoutée goutte à goutte à une solution de 2,47 g de xanthate d'éthyle potassium dans 5 ml d'eau à 70 °C. Le mélange réactionnel est dilué au moyen d'acétate d'éthyle. La phase organique est lavée au moyen d'une solution de soude 1N, d'une solution saturée de chlorure de sodium puis séchée sur sulfate de magnésium. Après évaporation des solvants, le résidu est repris dans 15 ml d'éthanol. On ajoute 1,47 g de potasse et on chauffe à 45 °C pendant 10 minutes. Le mélange réactionnel est ensuite versé sur une solution aqueuse glacée et partiellement purifié par extraction à l'acétate d'éthyle. La phase aqueuse, refroidie avec de la glace, est rendue acide par addition d'acide chlorhydrique concentré puis extraite à l'acétate d'éthyle. La phase organique est séchée sur sulfate de magnésium et les solvants sont évaporés à sec. On obtient ainsi 640 mg (rendement : 60 %) du produit attendu.
F = 116 °C

VII b) Obtention du 4-[(4-fluoro-phényl)sulfonyl]phényl 2,3,4-tri-O-acétyl-1,5-dithio-$\beta$-D-xylopyranoside - (exemple 13)

En opérant de façon analogue à la préparation II c) on obtient le produit attendu.
F = 80 °C
$[\alpha]_D^{21}$ = + 48,4 ° (c = 0,5 ; $CHCl_3$).

VII c) Obtention du 4-[(4-fluorophényl)sulfonyl]phényl 1,5-dithio-$\beta$-D-xylopyranoside (exemple 14)

En opérant de façon analogue à la préparation I c) on obtient le produit attendu.
F = 136-138 °C
$[\alpha]_D^{21}$ = + 40 ° (c = 0,5 ; diméthylsulfoxyde).

**PREPARATION VIII**

VIII a) Obtention du 4-[(4-méthoxyphényl)sulfonyl]-benzonitrile

Dans un mélange de 120 ml d'éthanol et 12 ml d'eau on ajoute 6 g (0,0248 mole) de 4-[(4-méthoxyphényl)thio]-benzonitrile puis 18,45 g de monoperoxyphtalate de magnésium hexahydraté. Le milieu réactionnel est maintenu à 40 °C pendant 20 minutes. Après hydrolyse sur de l'eau glacée, la solution est filtrée et le solide blanc est lavé à l'eau. On obtient ainsi 5,2 g (rendement : 77 %) du produit attendu.
F = 135 °C

VIII b) Obtention du 4-[(4-hydroxyphényl)sulfonyl]-benzonitrile

Un mélange de 5,12 g (0,0187 mole) de 4-[(4-méthoxyphényl)sulfonyl]benzonitrile et de 21,6 g de chlorhydrate de pyridinium est maintenu à 200 °C pendant 2 heures. Après refroidissement, le milieu réactionnel est hydrolysé sur une solution d'acide chlorhydrique 1N. Le précipité formé est filtré, rincé au moyen d'une solution d'acide chlorhydrique 1N puis au moyen d'eau jusqu'à pH neutre. On obtient ainsi 4,5 g (rendement : 99 %) du produit attendu sous forme de solide gris.
F = 177 °C

VIII c) Obtention du diméthylthiocarbamate de 0-[4-((4-cyanophényl)sulfonyl)phényle]

A une solution de 570 mg de potasse dans 35 ml d'eau on ajoute 2,36 g (0,0097 mole) de 4-[(4-hydroxyphényl)sulfonyl]-benzonitrile.La solution est maintenue 15 minutes à température ambiante. Après refroidissement à 0 °C on ajoute, goutte à goutte, une solution de 1,38 g de chlorure de N,N-diméthylthiocarbamoyle dans 35 ml d'acétone. Après 4 heures le mélange réactionnel est hydrolysé sur une solution d'acide chlorhydrique 1N. La solution est extraite à l'acétate d'éthyle. La phase organique est lavée à l'eau jusqu'à pH neutre, séchée sur sulfate de magnésium et concentrée à sec. On obtient ainsi 3 g (rendement : 100 %) du produit attendu.
F = 158-167 °C

VIII d) Obtention du diméthylthiocarbamate de S-[4-((4-cyanophényl)sulfonyl)phényl]

3 g (0,0096 mole) de diméthylthiocarbamate de O-[4-((4-cyanophényl)sulfonyl)phényle] sont maintenus à 200 °C pendant 30 minutes. Après chromatographie sur gel de silice en éluant au moyen d'un mélange toluène/acétate d'éthyle 8/2 (v/v) on obtient 2,29 g (rendement : 76 %) du produit attendu.
F = 140 °C

VIII e) Obtention du 4-[(4-mercaptophényl)sulfonyl]-benzonitrile

A une solution de 2,25 g (0,0075 mole) de diméthylthiocarbamate de S-[4-((4-cyanophényl) sulfonyl)-phényle] dans 45 ml de N,N-diméthylformamide à 0 °C, on ajoute 4,1 ml de méthanolate de sodium (8 % de Na (p/v) dans le méthanol). Le mélange réactionnel est ensuite hydrolysé sur une solution glacée d'acide chlorhydrique 1N. Après filtration, le précipité formé est lavé à l'eau puis séché. On obtient ainsi 1,54 g (rendement : 78 %) du produit attendu.

F = 166 °C

VIII f) Obtention du 4-((4-cyanophényl)sulfonyl)phényl 2,3,4-tri-O-acétyl-1,5-dithio-β-D-xylopyranoside - (exemple 15)

En opérant de façon analogue à la préparation I b) on obtient le produit attendu.
F = 194-195 °C
$[\alpha]_D^{20}$ = + 51 ° (c = 0,5 ; $CHCl_3$)

VIII g) Obtention du 4-((4-cyanophényl)sulfonyl)phényl 1,5-dithio-β-D-xylopyranoside (exemple 16)

En opérant de façon analogue à la préparation I c) on obtient le produit attendu.
F = 175-183 °C
$[\alpha]_D^{20}$ = + 58,6 ° (c = 0,5 diméthylsulfoxyde).

**PREPARATION IX**

IX a) Obtention du 4-(N,N-diméthylsulfonamidyl)phényl 2,3,4-tri-O-acétyl-1,5-dithio-β-D-xylopyranoside - (exemple 17)

En opérant de façon analogue à la préparation I b) on obtient le produit attendu.
F = 120 °C
$[\alpha]_D^{20}$ = + 35,4 ° (c = 0,56 ; $CHCl_3$)

IX b) Obtention du 4-(N,N-diméthylsulfonamidyl)phényl 1,5-dithio-β-D-xylopyranoside (exemple 18)

En opérant de façon analogue à la préparation I c) on obtient le produit attendu.
F = 208-213 °C
$[\alpha]_D^{24}$ = + 21,4 ° (c = 0,42 ; diméthylsulfoxyde).

**PREPARATION X**

X a) Obtention du 4-(N,N-diméthylsulfonamidyl)phényl 2,3,4-tri-O-acétyl-5-thio-β-D-xylopyranoside (exemple 19)

En opérant de façon analogue à la préparation IV a) on obtient le produit attendu.
F = 85 °C puis 164-167 °C (double point de fusion)
$[\alpha]_D^{25}$ = - 57,6 ° (c = 0,33 ; $CHCl_3$)

X b) Obtention du 4-(N,N-diméthylsulfonamidyl)phényl 5-thio-β-D-xylopyranoside (exemple 20)

En opérant de façon analogue à la préparation I c) on obtient le produit attendu.
F = 205 °C
$[\alpha]_D^{21}$ = - 70,4 ° (c = 0,27 ; méthanol)

**PREPARATION XI**

XI a) Obtention du 4-mercapto-N-(1-pipéridinyl)-benzénesulfonamide

En opérant de façon analogue à la préparation I a) on obtient le produit attendu.
F = 93 °C

XI b) Obtention du 4-(N-(1-pipéridinyl)sulfonamidyl) phényl 2,3,4-tri-O-acétyl-1,5-dithio-β-D-xylopyranoside - (exemple 21)

En opérant de façon analogue à la préparation I b) on obtient le produit attendu.
F = 175-180 °C
$[\alpha]_D^{22}$ = + 39,8 ° (c = 0,425 ; $CHCl_3$)

XI c) Obtention du 4-(N-(1-pipéridinyl)sulfonamidyl) phényl 1,5-dithio-β-D-xylopyranoside (exemple 22)

En opérant de façon analogue à la préparation I c) on obtient le produit attendu.
F = 145-149 °C
$[\alpha]_D^{22}$ = + 23,8 ° (c = 0,21 ; méthanol)

**PREPARATION XII**

XII a) Obtention du 4-mercapto-N-(1-morpholinyl)-benzènesulfonamide

En opérant de façon analogue à la préparation I a) on obtient le produit attendu.
F = 128 °C

XII b) Obtention du 4-(N-(1-morpholinyl)sulfonamidyl) phényl 2,3,4-tri-O-acétyl-1,5-dithio-β-D-xylopyranoside (exemple 23)

En opérant de façon analogue à la préparation I b) on obtient le produit attendu.
F = 120-123 °C
$[\alpha]_D^{22}$ = + 39 ° (c = 0,39 ; $CHCl_3$)

XII c) Obtention du 4-(N-(1-morpholinyl)sulfonamidyl) phényl 1,5-dithio-β-D-xylopyranoside (exemple 24)

En opérant de façon analogue à la préparation I c) on obtient le produit attendu.
F = 90-105 °C
$[\alpha]_D^{22}$ = + 20,4 ° (c = 0,56 ; diméthylsulfoxyde) De façon non limitative, on a consigné les composés suivant l'invention dans le tableau I donné ci-après.

L'activité anti-thrombotique des produits selon l'invention a été mise en évidence selon le protocole opératoire de thrombose veineuse suivant :

On réalise une stase veineuse sous hypercoagulation selon la technique décrite par WESSLER et al. (J. Applied Physiol. 1959, p. 943-946) L'agent hypercoagulant utilisé est, comme dans la technique décrite par J. HAUPMAN et al. (Thrombosis and Haemostasis 43 (2) 1980, p. 118), une solution de facteur X activé (Xa) fourni par la société dite Flow Laboratories (71 Knat pour 12,5 ml de sérum physiologique).

L'étude est réalisée sur des rats mâles Wistar, non à jeûn, de 250 à 280 g répartis en lots de 10 animaux chacun. Les produits à tester sont administrés per os, en suspension dans le PEG 400. Une thrombose est induite 4 heures après ce traitement et le thrombus formé est prélevé et pesé.

Les résultats obtenus à la dose de 3 mg/kg p.o. ont été consignés dans le tableau I. On a également consigné dans ce tableau les résultats obtenus avec les produits connus de l'art antérieur précité.

TABLEAU I

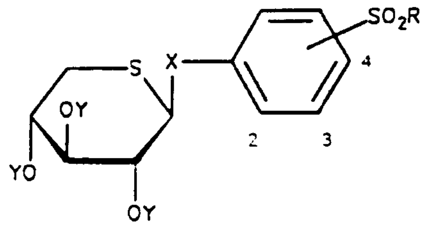

(I)

| Exemple | X | Position de $SO_2R$ | R | Y | % d'inhibition à 3 mg/kg |
|---|---|---|---|---|---|
| 1 | S | 4 | $-C_6H_5$ | $-COCH_3$ | 20 |
| 2 | S | 4 | $-C_6H_5$ | -H | 49 |
| 3 | S | 2 | $-CH_3$ | $-COCH_3$ | 29 |
| 4 | S | 2 | $-CH_3$ | -H | 26 |
| 5 | S | 4 | $-CH_3$ | $-COCH_3$ | - |
| 6 | S | 4 | $-CH_3$ | -H | 61 |
| 7 | O | 4 | $-CH_3$ | $-COCH_3$ | - |
| 8 | O | 4 | $-CH_3$ | -H | 30 |
| 9 | S | 4 | $-CH_2-CH_3$ | $-COCH_3$ | 31 |
| 10 | S | 4 | $-CH_2-CH_3$ | -H | 31 |
| 11 | S | 3 | $-CH_3$ | $-COCH_3$ | - |

TABLEAU I (suite 1)

| Exemple | X | Position de $SO_2R$ | R | Y | % d'inhibition à 3 mg/kg |
|---|---|---|---|---|---|
| 12 | S | 3 | $-CH_3$ | -H | 43 |
| 13 | S | 4 | $4-F-C_6H_5$ | $-COCH_3$ | 58 |
| 14 | S | 4 | $4-F-C_6H_5$ | -H | 61 |
| 15 | S | 4 | $4-CN-C_6H_5$ | $-COCH_3$ | - |
| 16 | S | 4 | $4-CN-C_6H_5$ | -H | 66 |
| 17 | S | 4 | $-N(CH_3)_2$ | $-COCH_3$ | 56 |
| 18 | S | 4 | $-N(CH_3)_2$ | -H | 46 |
| 19 | O | 4 | $-N(CH_3)_2$ | $-COCH_3$ | 23 |
| 20 | O | 4 | $-N(CH_3)_2$ | -H | 58 |

TABLEAU I (fin)

| Exemple | X | Position de $SO_2R$ | R | Y | % d'inhibition à 3 mg/kg |
|---|---|---|---|---|---|
| 21 | S | 4 | 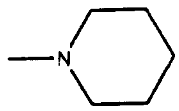 | $-COCH_3$ | - |
| 22 | S | 4 | 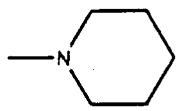 | -H | 47 |
| 23 | S | 4 | 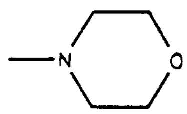 | $-COCH_3$ | - |
| 24 | S | 4 | 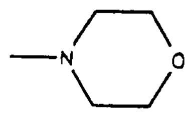 | -H | - |
| A | Produit de comparaison décrit à l'exemple 1 de EP-A-0 133 103 | | | | 14(1) |
| B | Produit de comparaison décrit à l'exemple 97 de EP-B-0 051 023 | | | | 5,5(1) |
| C | Produit de comparaison décrit à l'exemple 3 de EP-A-0 290 321 | | | | 20(2) |

Notes : (1) produit testé à 12,5 mg/kg p.o.
(2) produit testé à 3 mg/kg p.o.

## Revendications

**Revendications pour les Etats contractants suivants : AT, BE, CH, DE, DK, FR, GB, IT, LI, LU, NL, SE**

**1.** Composé oside caractérisé en ce qu'il est choisi parmi l'ensemble comprenant les sulfonyl-phényl-β-D-thioxylosides de formule :

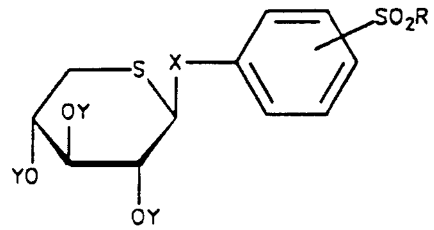

(I) .

dans laquelle :

- X représente un atome de soufre ou un atome d'oxygène,
- R représente un groupe alkyle en $C_1$-$C_4$, un groupe amino substitué $NR_1R_2$, où $R_1$ et $R_2$, identiques ou différents, représentent chacun un groupe alkyle en $C_1$-$C_4$, $R_1$ et $R_2$ considérés ensemble pouvant former avec l'atome d'azote auquel ils sont liés un groupe pipéridinyle ou morpholinyle, ou un groupe phényle éventuellement substitué en position para par un groupe cyano ou par un atome d'halogène; et,
- Y représente l'atome d'hydrogène ou un groupe acyle aliphatique.

**2.** Composé oside selon la revendication 1 caractérisé en ce que X représente l'atome de soufre.

**3.** Composé oside selon les revendications 1 ou 2, caractérisé en ce que le groupe acyle aliphatique Y comporte de 2 à 5 atomes de carbone et représente notamment le groupe $CH_3CO$.

**4.** Composition thérapeutique caractérisée en ce qu'elle renferme, en association avec un excipient physiologiquement acceptable, au moins un composé oside selon l'une quelconque des revendications 1 à 3.

**5.** Utilisation d'une substance selon l'une quelconque des revendications 1 à 3, pour l'obtention d'un médicament antithrombotique destiné à une utilisation thérapeutique vis-à-vis des troubles de la circulation veineuse.

**6.** Procédé de préparation d'un sulfonyl-phényl-$\beta$-D-thioxyloside de formule I selon la revendication 1 caractérisé en ce que :

(i) on fait réagir un composé de formule :

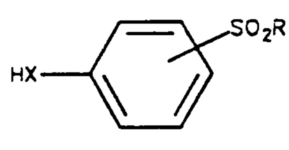

(II)

où X et R sont définis comme ci-dessus,

avec un dérivé du thioxylose choisi parmi l'ensemble comprenant :

(i) les halogénures d'acylthioxylosyle de formule :

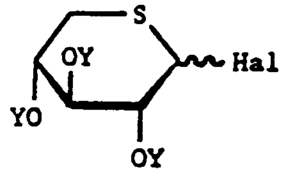

(III)

(ii) les thioxyloses peracylés de formule :

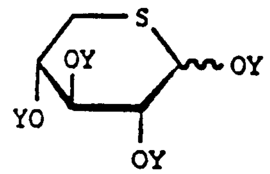 (IV)

et,
(iii) les trichloracétimidates d'acylthioxylosyle de formule :

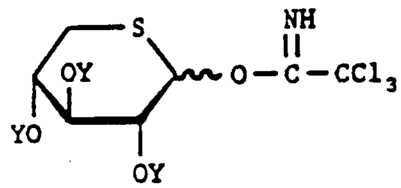 (V)

dans lesquelles Hal représente un atome d'halogène tel que Cl ou Br, l'atome de brome étant ici l'atome d'halogène préféré, et Y représente un groupe acyle, notamment un groupe acyle aliphatique comprenant un nombre total d'atomes de carbone de 2 à 5 et étant de préférence le troupe acétyle,
dans un solvant inerte, à raison de 1 mole de II pour environ 0,6 à 1,2 moles de composé III, IV ou V, en présence d'un accepteur d'acide et/ou d'un acide de Lewis et,
(ii) si nécessaire, on soumet le composé de formule I ainsi obtenu où Y est un groupe acyle aliphatique en $C_2$-$C_5$ à une réaction de désacylation à une température comprise entre 0 °C et la température de reflux du milieu réactionnel, dans un alcool inférieur en $C_1$-$C_4$, de préférence le méthanol, en présence d'un alcoolate métallique, de préférence le méthylate de magnésium ou le méthylate de sodium, pour obtenir un composé de formule I où Y est H.

**7.** Procédé selon la revendication 6, caractérisé en outre par le fait que le composé de formule II où X représente l'atome de soufre intervenant au stade (i) est préparé selon les étapes suivantes :
a) condensation, en milieu basique fort du chlorure de diméthylaminothiocarbamoyle de formule :

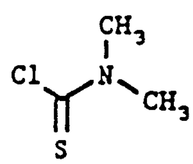 (VI)

avec un composé de formule :

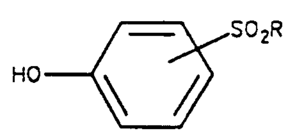 (IIa)

où $R_1$ et $R_2$ ont les significations indiquées ci-dessus, pour obtenir un composé de formule :

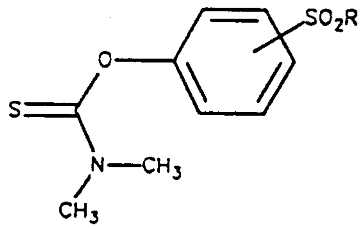

(VII)

où $R_1$ et $R_2$ sont définis comme ci-dessus,

b) réarrangement du composé de formule VII ainsi obtenu par chauffage, pour obtenir un composé de formule :

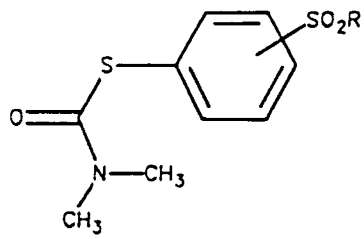

(VIII)

où $R_1$ et $R_2$ sont définis comme indiqués ci-dessus et,

c) traitement du composé de formule VIII ainsi obtenu par un alcoolate métallique, de préférence le méthanolate de sodium ou de magnésium, dans un alcool inférieur en $C_1$-$C_4$, le diméthylformamide ou le dioxanne, pour obtenir un composé de formule II où X = S.

**8.** Produit intermédiaire nouveau intervenant dans la synthèse des sulfonyl-phényl-β-D-thioxylosides de formule I où X représente l'atome de soufre selon la revendication 2, caractérisé en ce qu'il est choisi parmi l'ensemble constitué par les composés de formule :

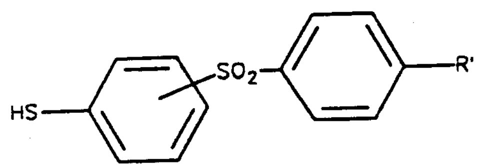

dans laquelle :

- R′ représente un atome de fluor, un atome de brome ou un groupe cyano.

**Revendications pour les Etats contractants suivants : ES, GR**

**1.** Procédé de préparation d'un sulfonyl-phényl-β-D-thioxyloside de formule I :

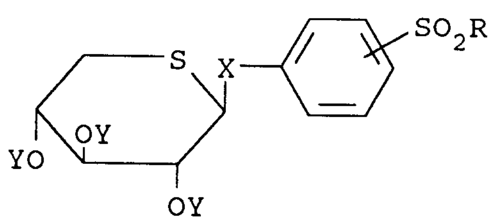

(I)

dans laquelle :

- X représente un atome de soufre ou un atome d'oxygène,

- R représente un groupe alkyle en $C_1$-$C_4$, un groupe amino substitué $NR_1R_2$, où $R_1$ et $R_2$, identiques ou différents, représentent chacun un groupe alkyle en $C_1$-$C_4$, $R_1$ et $R_2$ considérés ensemble pouvant former avec l'atome d'azote auquel ils sont liés un groupe pipéridinyle ou morpholinyle, ou un groupe phényle éventuellement substitué en position para par un groupe cyano ou par un atome d'halogène ; et,
- Y représente l'atome d'hydrogène ou un groupe acyle aliphatique ;

ledit procédé étant caractérisé en ce que :

(i) on fait réagir un composé de formule :

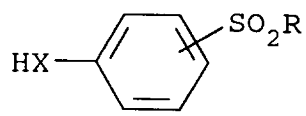

(II)

où X et R sont définis comme ci-dessus, avec un dérivé du thioxylose choisi parmi l'ensemble comprenant :

(i) les halogénures d'acylthioxylosyle de formule :

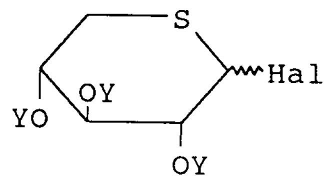

(III)

(ii) les thioxyloses peracylés de formule :

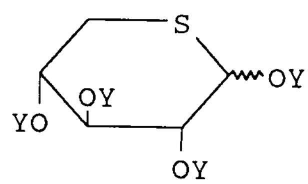

(IV)

et,

(iii) les trichloracétimidates d'acylthioxylosyle de formule :

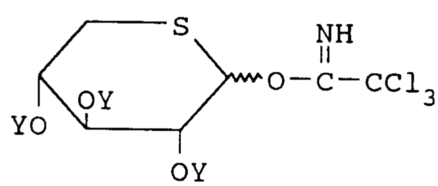

(V)

dans lesquelles Hal représente un atome d'halogène tel que Cl ou Br, l'atome de brome étant ici l'atome d'halogène préféré, et Y représente un groupe acyle, notamment un groupe acyle aliphatique comprenant un nombre total d'atomes de carbone de 2 à 5 et étant de préférence le groupe acétyle,

dans un solvant inerte, à raison de 1 mole de II pour environ 0,6 à 1,2 moles de composé III, IV ou V, en présence d'un accepteur d'acide et/ou d'un acide de Lewis, et

(ii) si nécessaire, on soumet le composé de formule I ainsi obtenu où Y est un groupe acyle aliphatique en $C_2$-$C_5$ à une réaction de désacylation à une température comprise entre 0 °C et la température de reflux du milieu réactionnel, dans un alcool inférieur en $C_1$-$C_4$, de préférence le méthanol, en présence d'un alcoolate métallique, de préférence le méthylate de magnésium ou le méthylate de sodium, pour obtenir un composé de formule I ou Y est H.

2. Procédé selon la revendication 1, caractérisé en outre par le fait que le composé de formule II où X représente l' atome de soufre intervenant au stade (i) est préparé selon les étapes suivantes :

a) condensation, en milieu basique fort du chlorure de diméthylaminothiocarbamoyle de formule :

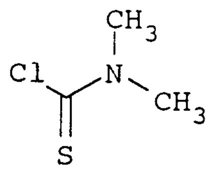

(VI)

avec un composé de formule :

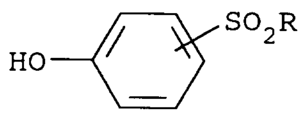

(IIa)

où $R_1$ et $R_2$ ont les significations indiquées ci-dessus, pour obtenir un composé de formule :

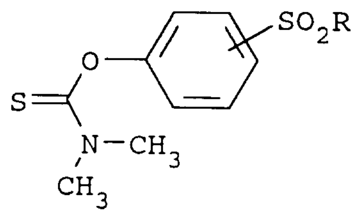

(VII)

où $R_1$ et $R_2$ sont définis comme ci-dessus,

b) réarrangement du composé de formule VII ainsi obtenu par chauffage, pour obtenir un composé de formule :

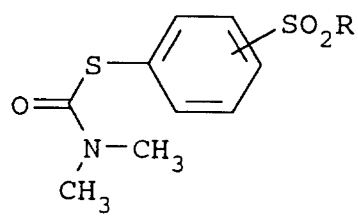

(VIII)

où $R_1$ et $R_2$ sont définis comme indiqué ci-dessus et,

c) traitement du composé de formule VIII ainsi obtenu par un alcoolate métallique, de préférence le méthanolate de sodium ou de magnésium, dans un alcool inférieur en $C_1$-$C_4$, le diméthylformamide ou le dioxanne, pour obtenir un composé de formule II où X = S.

## Claims

**Claims for the following Contracting States : AT, BE, CH, DE, DK, FR, GB, IT, LI, LU, NL, SE**

1. An oside compound selected from the group consisting of the sulfonylphenyl-$\beta$-D-thioxylosides of the formula

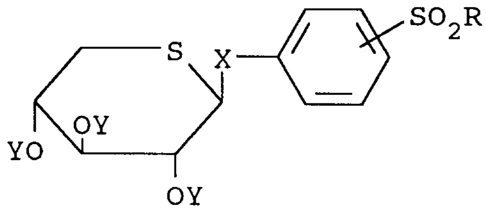

(I)

in which :

- X is a sulfur atom or an oxygen atom ;
- R is a $C_1$-$C_4$ alkyl group, a substituted amino group $NR_1R_2$, wherein $R_1$ and $R_2$, which are identical or different, are each a $C_1$-$C_4$ alkyl group, it being possible for $R_1$ and $R_2$, taken together, to form a piperidinyl or morpholinyl group with the nitrogen atom to which they are bonded, or a phenyl group which is unsubstituted or substituted in the para position by a cyano group or by a halogen atom ; and,
- Y is the hydrogen atom or an aliphatic acyl group.

**2.** An oside compound according to claim 1, wherein X is the sulfur atom.

**3.** An oside compound according to claim 1 or claim 2, wherein the aliphatic acyl group Y contains from 2 to 5 carbon atoms and is especially the group $CH_3CO$.

**4.** A therapeutic composition containing, in association with a physiologically acceptable excipient, at least one oside compound according to any one of claims 1 to 3.

**5.** A use of an oside compound of the formula I according to any one of claims 1 to 3 for the obtention of an antithrombotic drug destined to a therapeutical use vis-a-vis disorders of the venous circulation.

**6.** A method of preparing a sulfonylphenyl-$\beta$-D-thioxyloside of formula I according to claim 1, which comprises

(i) reacting a compound of the formula

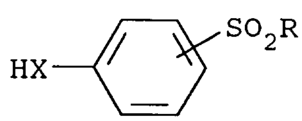

(II)

in which X and R are as defined above, with a thioxylose derivative selected from the group consisting of

(i) the acylthioxylosyl halides of the formula

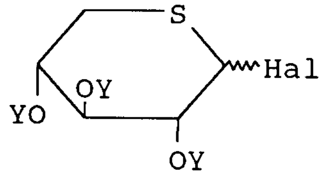

(III)

(ii) the peracylated thioxyloses of the formula

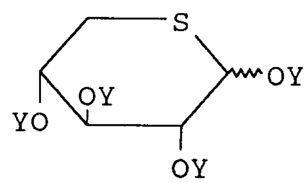

(IV)

and

(iii) the acylthioxylosyl trichloroacetimidates of the formula

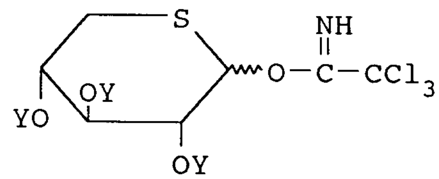

(V)

in which Hal is a halogen atom such as Cl or Br, the bromine atom being the preferred halogen atom here, and Y is an acyl group, especially an aliphatic acyl group containing a total of 2 to 5 carbon atoms and preferably the acetyl group,

in an inert solvent, at a rate of 1 mol of II to about 0.6 to 1.2 mol of compound III, IV or V, in the presence of an acid acceptor and/or a Lewis acid, and

(ii) if necessary, subjecting the resulting compound of formula I in which Y is a $C_2$-$C_5$ aliphatic acyl group to a deacylation reaction at a temperature of between 0°C and the reflux temperature of the reaction medium, in a $C_1$-$C_4$ lower alcohol, preferably methanol, in the presence of a metal alcoholate, preferably magnesium methylate or sodium methylate, to give a compound of formula I in which Y is H.

**7.** A method according to claim 6, which is further characterized by the fact that the compound of formula II in which X is the sulfur atom, involved in stage (i), is prepared according to the following steps :

a) condensation of dimethylaminothiocarbamoyl chloride of the formula

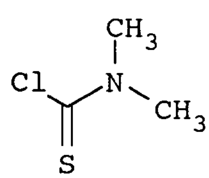

(VI)

in a strong basic medium with a compound of the formula

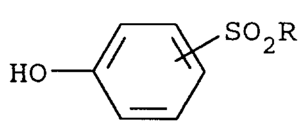

(IIa)

in which $R_1$ and $R_2$ are as defined above, to give a compound of the formula

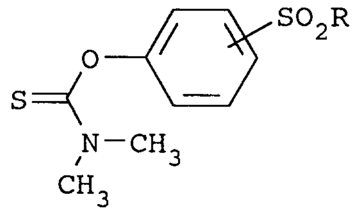

(VII)

in which $R_1$ and $R_2$ are as defined above,

b) rearrangement of the resulting compound of formula VII, by heating, to give a compound of the formula

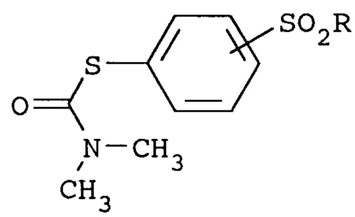

(VIII)

in which $R_1$ and $R_2$ are as defined above, and

c) treatment of the resulting compound of formula VIII with a metal alcoholate, preferably sodium or magnesium methylate, in a $C_1$-$C_4$ lower alcohol, dimethylformamide or dioxane, to give a compound of formula II in which X = S.

**8.** A novel intermediate compound involved in the synthesis of the sulfonylphenyl-β-D-thioxylosides of formula I in which X is the sulfur atom, according to claim 2, selected from the group consisting of the compounds of the formula

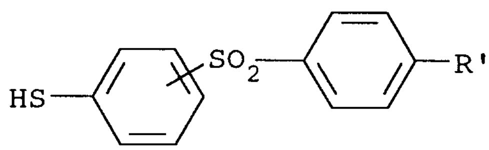

in which :

- R' is a fluorine atom, a bromine atom or a cyano group.

**Claims for the following Contracting States : ES, GR**

**1.** A method of preparing a sulfonylphenyl-β-D-thioxyloside of the formula

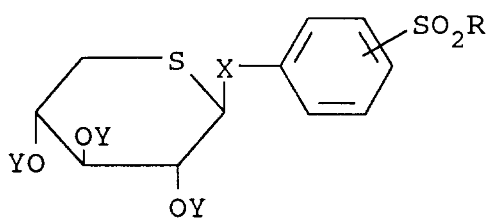

(I)

in which :

- X is a sulfur atom or an oxygen atom ;
- R is a $C_1$-$C_4$ alkyl group, a substituted amino group $NR_1R_2$, wherein $R_1$ and $R_2$, which are identical of different, are each a $C_1$-$C_4$ alkyl group, it being possible for $R_1$ and $R_2$, taken

together, to form a piperidinyl or morpholinyl group with the nitrogen atom to which they are bonded, or a phenyl group which is unsubstituted or substituted in the para position by a cyano group or by a halogen atom ; and,

- Y is the hydrogen atom or an aliphatic acyl group, said method comprising

(i) reacting a compound of the formula

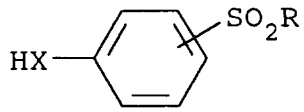

(II)

in which X and R are as defined above,
with a thioxyloside derivative selected from the group consisting of

(i) the acylthioxylosyl halides of the formula

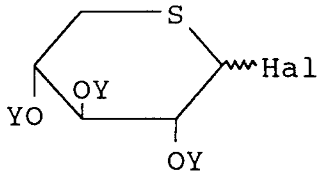

(III)

(ii) the peracylated thioxyloses of the formula :

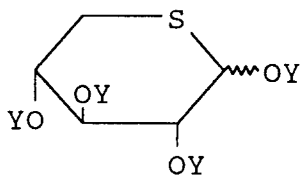

(IV)

and

(iii) the acylthioxylosyl trichloroacetimidates of the formula

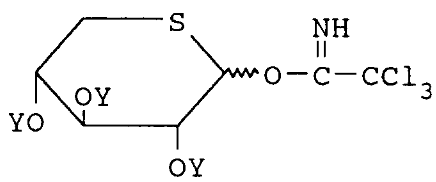

(V)

in which Hal is a halogen atom such as Cl or Br, the bromine atom being the preferred halogen atom here, and Y is an acyl group, especially an aliphatic acyl group containing a total of 2 to 5 carbon atoms and preferably the acetyl group,

in an inert solvant, at a rate of 1 mol of II to about 0.6 to 1.2 mol of compound III, IV or V, in the presence of an acid acceptor and/or a Lewis acid, and

(ii) if necessary, subjecting the resulting compound of formula I in which Y is a $C_2$-$C_5$ aliphatic acyl group to a deacylation reaction at a temperature of between 0 °C and the reflux temperature of the reaction medium, in a $C_1$-$C_4$ lower alcohol, preferably methanol, in the presence of a metal alcoholate, preferably magnesium methylate or sodium methylate, to give a compound of fromula I in which Y is H.

**2.** A method according to claim 1, which is further characterized in that the compound of formula II in which X is the sulfur atom, involved in stage (i), is prepared according to the following steps :

a) condensation of dimethylaminothiocarbamoyl chloride of the formula

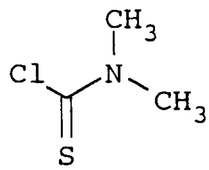 (VI)

in a strong basic medium with a compound of the formula

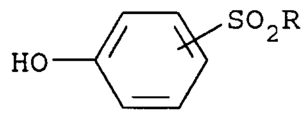 (IIa)

in which $R_1$ and $R_2$ are as defined above, to give a compound of the formula

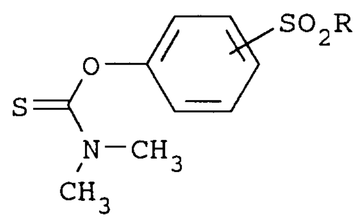 (VII)

in which $R_1$ and $R_2$ are as defined above,

b) rearrangement of the resulting compound of formula VII, by heating, to give a compound of the formula

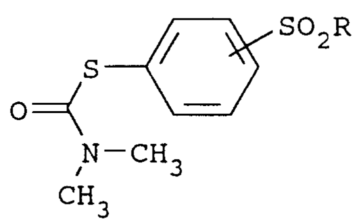 (VIII)

in which $R_1$ and $R_2$ are as defined above, and

c) treatment of the resulting compound of formula VIII with a metal alcoholate, preferably sodium or magnesium methylate, in a $C_1$-$C_4$ lower alcohol, dimethylformamide or dioxane, to give a compound of formula II in which X = S.

**Patentansprüche**
**Patentansprüche für folgende Vertragsstaaten : AT, BE, CH, DE, DK, FR, GB, IT, LI, LU, NL, SE**

1. Osid-Verbindung, dadurch gekennzeichnet, daß sie aus der Gruppe, bestehend aus den Sulphonylphenyl-$\beta$-D-thioxylosiden der Formel

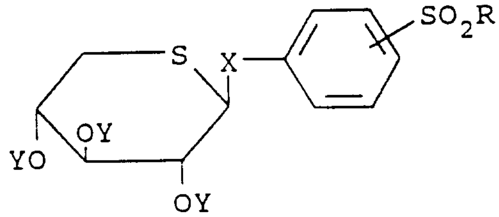

(I)

wobei

- X ein Schwefel- oder ein Sauerstoffatom darstellt,
- R einen $C_1$-$C_4$-Alkylrest, einen substituierten Aminorest $NR_1R_2$, bei dem $R_1$ und $R_2$ gleich oder verschieden sind, wobei jeder einen $C_1$-$C_4$-Alkylrest darstellt, und die Reste $R_1$ und $R_2$ gemeinsam mit einem Stickstoffatom, an welches sie gebunden sind, eine Piperidinyl- oder Morpholinylgruppe bilden können, oder eine gegebenenfalls an der para-Position durch eine Cyanogruppe oder ein Halogenatom substituierte Phenylgruppe bedeutet,
  und
- Y ein Wasserstoffatom oder einen aliphatischen Acylrest darstellt, gewählt wird.

**2.** Osid-Verbindung nach Anspruch 1, dadurch gekennzeichnet, daß X ein Schwefelatom darstellt.

**3.** Osid-Verbindung nach den Ansprüchen 1 oder 2, dadurch gekennzeichnet, daß der aliphatische Acylrest Y 2 bis 5 Kohlenstoffatome umfaßt, und insbesondere die $CH_3CO$-Gruppe darstellt.

**4.** Arzneimittel, dadurch gekennzeichnet, daß es in Kombination mit einem physiologisch verträglichen Exzipienten wenigstens eine Osid-Verbindung nach einem der Ansprüche 1 bis 3 umfaßt.

**5.** Verwendung einer Substanz nach einem der Ansprüche 1 bis 3, zur Herstellung eines antithrombotischen Medikaments, das für die therapeutische Verwendung bei venösen Kreislaufstörungen bestimmt ist.

**6.** Verfahren zur Herstellung eines Sulphonylphenyl-$\beta$-D-thioxylosids der Formel I nach Anspruch 1, dadurch gekennzeichnet, daß

(i) man eine Verbindung der Formel

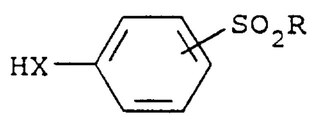

(II)

wobei X und R wie vorstehend definiert sind,

mit einem Thioxylose-Derivat, gewählt aus der Gruppe, bestehend aus

(i) den Acylthioxylosylhalogeniden der Formel

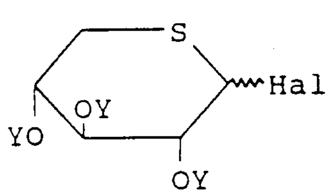

(III)

(ii) den peracylierten Thioxylosen der Formel

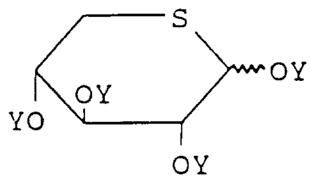

(IV)

und
(iii) den Acylthioxylosyl-trichloracetimidaten der Formel

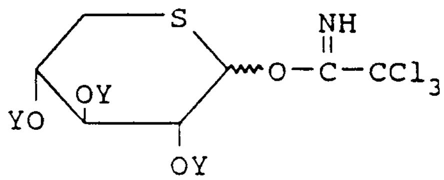

(V)

wobei Hal ein Halogenatom wie Cl oder Br bedeutet, wobei hier das Bromatom das bevorzugte Halogenatom ist, und Y einen Acylrest darstellt, insbesondere einen aliphatischen Acylrest, umfassend eine Gesamtzahl an Kohlenstoffatomen von 2 bis 5, wobei die Acetylgruppe bevorzugt wird,

in einem inerten Lösemittel in einem Verhältnis von 1 Mol der Verbindung II zu ungefähr 0,6 bis 1,2 Mol der Verbindung III, IV oder V in Gegenwart eines Säureakzeptors und/oder einer Lewis-Säure umsetzt, und

(ii) man gegebenenfalls mit der so erhaltenen Verbindung der Formel I, wobei Y einen aliphatischen $C_2$-$C_5$-Acylrest bedeutet, bei einer Temperatur zwischen 0 °C und der Rückflußtemperatur des Reaktionsmediums in einem niederen $C_1$-$C_4$-Alkohol, vorzugsweise Methanol, in Gegenwart eines Metallalkoholats, vorzugsweise Magnesiummethylat oder Natriummethylat, eine Deacylierungsreaktion durchführt, um eine Verbindung der Formel I, wobei Y ein Wasserstoffatom darstellt, zu erhalten.

**7.** Verfahren nach Anspruch 6, unter anderem dadurch gekennzeichnet, daß die Verbindung der Formel II, wobei X ein Schwefelatom darstellt, die in Schritt (i) eingebracht wird, nach einem der folgenden Schritte hergestellt wird:

a) Kondensation des Dimethylthiocarbamoylchlorids der Formel

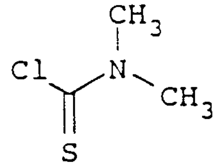

(VI)

in einem stark basischen Medium mit einer Verbindung der Formel

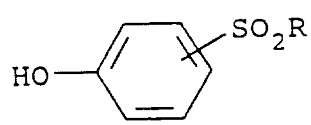

(IIa)

wobei $R_1$ und $R_2$ die vorstehend angegebenen Bedeutungen besitzen, um eine Verbindung der Formel

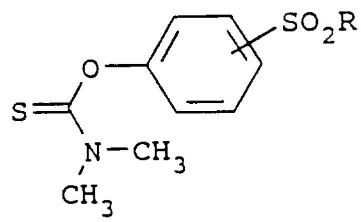

(VII)

wobei $R_1$ und $R_2$ wie vorstehend definiert sind, zu erhalten,

b) Umlagerung der so erhaltenen Verbindung der Formel VII durch Erhitzen, um eine Verbindung der Formel

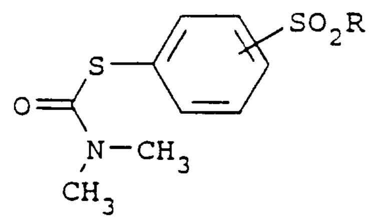

(VIII)

wobei $R_1$ und $R_2$ wie vorstehend angegeben definiert sind, zu erhalten, und

c) Behandeln der so erhaltenen Verbindung der Formel VIII mit einem Metallalkoholat, vorzugsweise dem Natrium- oder Magnesiummethanolat, in einem niederen $C_1$-$C_4$-Alkohol, Dimethylformamid oder Dioxan,

um eine Verbindung der Formel II, wobei X ein Schwefelatom ist, zu erhalten.

**8.** Neues Zwischenprodukt, das bei der Synthese der Sulphonylphenyl-$\beta$-D-thioxylosiden der Formel I, wobei X ein Schwefelatom nach Anspruch 2darstellt, mitwirkt, dadurch gekennzeichnet, daß es aus der Gruppe, bestehend aus den Verbindungen der Formel

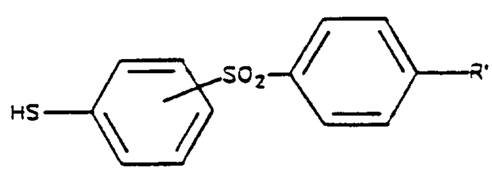

wobei R' ein Fluor- oder Bromatom oder eine Cyanogruppe bedeutet, gewählt wird.

**Patentansprüche für folgende Vertragsstaaten : ES, GR**

**1.** Verfahren zur Herstellung eines Sulphonylphenyl-$\beta$-D-thioxylosids der Formel I

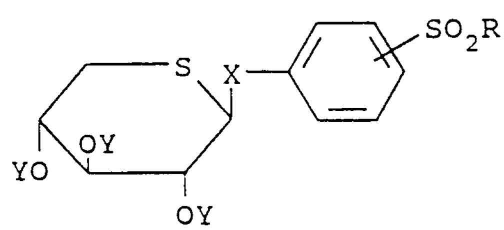

(I)

wobei

- X ein Schwefel- oder ein Sauerstoffatom darstellt,

- R einen $C_1$-$C_4$-Alkylrest, einen substituierten Aminorest $NR_1R_2$, bei dem $R_1$ und $R_2$ gleich oder verschieden sind, wobei jeder einen $C_1$-$C_4$-Alkyrest darstellt, und die Reste $R_1$ und $R_2$ gemeinsam mit einem Stickstoffatom, an welches sie gebunden sind, eine Piperidinyl- oder Morpholinylgruppe bilden können, oder eine gegebenenfalls an der para-Position durch eine Cyanogruppe oder ein Halogenatom substituierte Phenylgruppe bedeutet,
  und
- Y ein Wasserstoffatom oder einen aliphatischen Acylrest darstellt, wobei das genannte Verfahren dadurch gekennzeichnet ist, daß

(i) man eine Verbindung der Formel

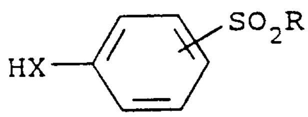

(II)

wobei X und R wie vorstehend definiert sind,
mit einem Thioxylose-Derivat, gewählt aus der Gruppe, bestehend aus

(i) den Acylthioxylosylhalogeniden der Formel

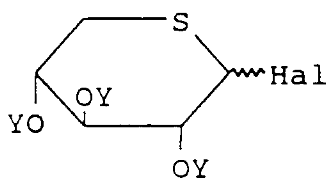

(III)

(ii) den peracylierten Thioxylosen der Formel

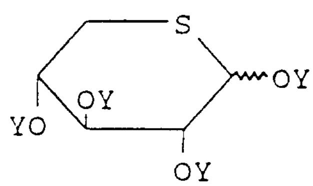

(IV)

und

(iii) den Acylthioxylosyl-trichloracetimidaten der Formel

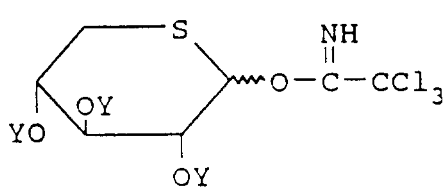

(V)

wobei Hal ein Halogenatom wie Cl oder Br bedeutet, wobei hier das Bromatom das bevorzugte Halogenatom ist, und Y einen Acylrest darstellt, insbesondere einen aliphatischen Acylrest, umfassend eine Gesamtzahl an Kohlenstoffatomen von 2 bis 5, wobei die Acetylgruppe bevorzugt wird,

in einem inerten Lösemittel in einem Verhältnis von 1 Mol der Verbindung II zu ungefähr 0,6 bis 1,2 Mol der Verbindung III, IV oder V in Gegenwart eines Säureakzeptors und/oder einer Lewis-Säure umsetzt,
und
(ii) man gegebenenfalls mit der so erhaltenen Verbindung der Formel I, wobei Y einen aliphatischen $C_2$-$C_5$-Acylrest bedeutet, bei einer Temperatur zwischen 0 °C und der Rückflußtemperatur des

Reaktionsmediums in einem niederen $C_1$-$C_4$-Alkohol, vorzugsweise Methanol, in Gegenwart eines Metallalkoholats, vorzugsweise Magnesiummethylat oder Natriummethylat, eine Deacylierungsreaktion durchführt, um eine Verbindung der Formel I, wobei Y ein Wasserstoffatom darstellt, zu erhalten.

**2.** Verfahren nach Anspruch 1, unter anderem dadurch gekennzeichnet, daß die Verbindung der Formel II, wobei X ein Schwefelatom darstellt, die in Schritt (i) eingebracht wird, nach einem der folgenden Schritte hergestellt wird:

a) Kondensation des Dimethylthiocarbamoylchlorids der Formel

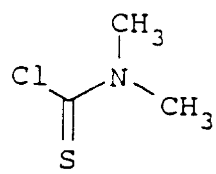

(VI)

in einem stark basischen Medium mit einer Verbindung der Formel

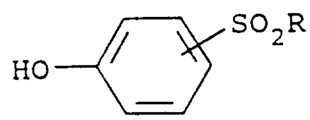

(IIa)

wobei $R_1$ und $R_2$ die vorstehend angegebenen Bedeutungen besitzen, um eine Verbindung der Formel

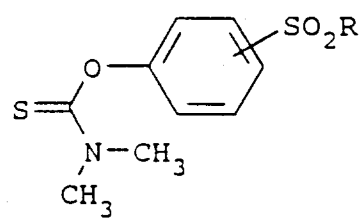

(VII)

wobei $R_1$ und $R_2$ wie vorstehend definiert sind,
zu erhalten,

b) Umlagerung der so erhaltenen Verbindung der Formel VII durch Erhitzen, um eine Verbindung der Formel

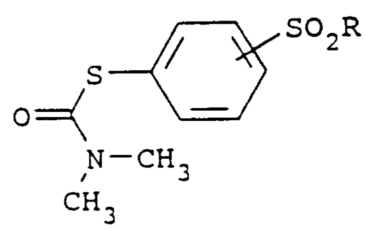

(VIII)

wobei $R_1$ und $R_2$ wie vorstehend angegeben definiert sind,
zu erhalten,
und

c) Behandeln der so erhaltenen Verbindung der Formel VIII mit einem Metallalkoholat, vorzugsweise dem Natrium- oder Magnesiummethanolat, in einem niederen $C_1$-$C_4$-Alkohol, Dimethylformamid oder Dioxan,
um eine Verbindung der Formel II, wobei X ein Schwefelatom ist, zu erhalten.